# EUROPEAN PATENT APPLICATION

(11) **EP 4 566 610 A1**
(43) Date of publication of application: **11.06.2025**
(21) Application number: 23383252.6
(22) Date of filing: 05.12.2023
(51) Int. Cl.: A61K 31/505, A61P 3/00, A61P 9/00, A61P 25/00, A61P 25/16, A61P 27/02, A61P 39/00, A61P 43/00

(54) **SENOLYTIC COMPOUNDS**

(71) Applicant: Universidade de Santiago de Compostela, 15782 Santiago de Compostela (ES); Servizo Galego de Saude, 15703 Santiago de Compostela (ES)
(72) Inventor: COLLADO RODRÍGUEZ, Manuel, 15703 Santiago de Compostela (ES); PRADO SÁNCHEZ, Miguel Ángel, 15782 Santiago de Compostela (ES)
(74) Representative: Hoffmann Eitle

(57) **Abstract**

The present invention refers to specific compounds for use as senolytic agents to selectively induce death of senescent cells, in the treatment and/or prevention of senescence-associated diseases or disorders.

## Description

### FIELD OF THE INVENTION

The present invention refers to the medical field. Particularly, the present invention refers to specific compounds for use as senolytic agents to selectively induce death of senescent cells, in the treatment and/or prevention of senescence-associated diseases or disorders.

### STATE OF THE ART

Senescence is a cellular programme that imposes a stable arrest on damaged or old cells to avoid their replication. As well as growth arrest, senescent cells undergo profound phenotypic changes that include chromatin reorganisation, increase of 3-galactosidase activity (referred to as senescence-associated β-galactosidase or SA-β-Gal) and secretion of multiple factors, mainly pro-inflammatory, that are collectively referred to as the senescence-associated secretory phenotype (SASP).

Replicative senescence is activated upon serial passage of cells in culture (or as cells become older in an organism). Senescence can also be induced by a range of different insults that include oncogene activation, irradiation and exposure to chemotherapeutic drugs.

Senescent cells accumulate during age and are associated with many diseases, including cancer, fibrosis and many age-related pathologies. Recent evidence suggests that senescent cells are detrimental in multiple pathologies and their elimination confers many advantages, ameliorating multiple pathologies and increasing healthspan and lifespan.

Senescent cells are present in many pre-neoplastic lesions, fibrotic tissues (e.g., in the liver, kidney, heart, pancreas) and old tissues. Senescent cells are also associated with a long list of other pathologies, including neurological (e.g. brain aneurysm, Alzheimer's and Parkinson), pulmonary (e.g. idiopathic pulmonary fibrosis, chronic obstructive pulmonary disease and cystic fibrosis), ophthalmological (e.g. cataracts, glaucoma, macular degeneration), musculoskeletal (e.g. sarcopenia, disc degeneration, osteoarthritis), cardiovascular (e.g. atherosclerosis, cardiac fibrosis, aorta aneurysm), renal (e.g. kidney disease, transplant complications) and others such as diabetes, mucositis, hypertension and osteomyelofibrosis (OMF).

While senescent cells have a protective role against cancer and limit most types of fibrosis, the accumulation of senescent cells during aging and many other diseases is thought to be detrimental. To date, there is no unequivocal explanation of why senescent cells are detrimental.

Evidence for the many detrimental effects of senescent cells (and the benefits caused by their selective elimination) has been provided by many authors, and proof-of-concept studies have led to the identification of compounds that can selectively eliminate senescent cells (so-called "senolytics"), most of them having significant side-effects.

So, there is an unmet medical need of identifying further compounds with senolytic properties.

### DESCRIPTION OF THE INVENTION

### Brief description of the invention

The present invention refers to specific compounds for use as senolytic agents or as senotherapeutic agents designed to selectively remove senescent cells and/or targeting the SASP.

More specifically, the senotherapeutic agent is a molecule able to either i) reduce the accumulation of senescent cells for example by inducing their death via apoptosis or inhibition of the SASP component and ii) trigger the re-differentiation of cells once they have been de-differentiated. Both reduced levels of senescence and enhancement of re-differentiation restore the capacity of the tissues to restore regeneration. The purpose of the "senotherapeutic agents" is to delay, prevent, alleviate, or reverse age-related diseases which are caused by the persistence and accumulation of senescent cells as senescence via SASP also induces inflammation, reprogramming and dedifferentiation of neighbouring cells, being therefore one of the key factors involved in tissue degeneration and progression of aging-associated diseases.

So, the compound of the invention selectively induces death of senescent cells and can be used in the treatment and/or prevention of senescence-associated diseases or disorders.

Particularly, the compound 96558 of *Formula I* was assayed in the present invention.

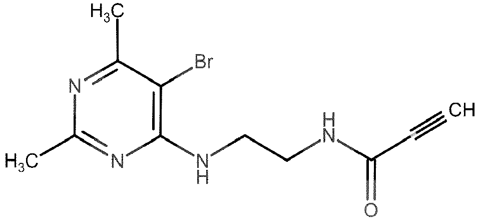

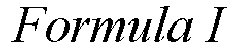

Such as it can be seen in **Example 2,** compound 96558 was validated as a senolytic agent in A549 bleomycin-induced senescent cells (**Example 2.2**) and under ionizing radiation-induced senescence (**Example 2.3**). Moreover, compound 96558 was explored in a different human tumor context, such as the U87MG glioma cell line (**Example 2.4**) and in a cellular aging model (**Example 2.5**)**,** confirming that the senolytic effect of compound 96558 is mediated by the activation of the apoptotic signalling pathway (**Example 2.6**).

So, the proof of concept shown in **Example 2** makes plausible the use the compound 96558 in the treatment and/or prevention of any senescence-associated disease or disorder [Chaib, S., Tchkonia, T., & Kirkland, J. L. (2022). Cellular senescence and senolytics: the path to the clinic. Nature medicine, 28(8), 1556-1568. https://doi.org/10.1038/s41591-022-01923-y]*.*

So, the first embodiment of the present invention refers to the compound of *Formula I,* or salts or derivatives thereof, for use as a medicament.

In a preferred embodiment, the present invention refers to the compound of *Formula I,* or salts or derivatives thereof, for use as a senolytic agent to selectively induce death of senescent cells.

In a preferred embodiment, the present invention refers to the compound of *Formula I,* or salts or derivatives thereof, for use in the treatment and/or prevention of senescence-associated diseases or disorders.

In a preferred embodiment, the present invention refers to the compound of *Formula I,* or salts or derivatives thereof, for use in the treatment and/or prevention of senescence-associated diseases or disorders selected from: cardiovascular disorder, metabolic disease, an inflammatory disease or disorder, a pulmonary disease or disorder, a neurological disease or disorder, a proliferative disorder, a renal disorder or disease, an eye disease or disorder, and/or a dermatological disorder or disease or cancer.

In a preferred embodiment, the present invention refers to the compound of *Formula I,* or salts or derivatives thereof, for use in the treatment and/or prevention of senescence-associated diseases or disorders selected from: (a) an inflammatory or autoimmune disease or disorder selected from osteoarthritis, osteoporosis, oral mucositis, inflammatory bowel disease, kyphosis or herniated intervertebral disc; (b) a neurological disease or disorder selected from Alzheimer's disease, Parkinson's disease, Huntington's disease, dementia, mild cognitive impairment, macular degeneration or motor neuron dysfunction; (c) a metabolic disease selected from diabetes, diabetic ulcer, metabolic syndrome or obesity; (d) a pulmonary disease selected from pulmonary fibrosis, chronic obstructive pulmonary disease, asthma, cystic fibrosis, emphysema, bronchiectasis or age-related loss of pulmonary function; (e) an eye disease or disorder selected from macular degeneration, glaucoma, cataracts, presbyopia or vision loss; (f) an age-related disorder selected from renal disease, renal failure, frailty, hearing loss, muscle fatigue, skin conditions, skin wound healing, liver fibrosis, pancreatic fibrosis, oral submucosa fibrosis or sarcopenia; (g) a dermatological disease or disorder selected from eczema, psoriasis, hyperpigmentation, nevi, rashes, atopic dermatitis, urticaria, diseases and disorders related to photosensitivity or photoaging, rhytides, pruritis, dysesthesia, eczematous eruptions, eosinophilic dermatosis, reactive neutrophilic dermatosis, pemphigus, pemphigoid, immunobullous dermatosis, fibrohistocytic proliferations of skin, cutaneous lymphomas or cutaneous lupus; or (h) a cardiovascular disease selected from atherosclerosis, angina, arrhythmia, cardiomyopathy, congestive heart failure, coronary artery disease, carotid artery disease, endocarditis, coronary thrombosis, myocardial infarction, hypertension, aortic aneurysm, cardiac diastolic dysfunction, hypercholesterolemia, hyperlipidemia, mitral valve prolapse, peripheral vascular disease, cardiac stress resistance, cardiac fibrosis, brain aneurysm or stroke.

The second embodiment of the present invention refers to a pharmaceutical composition comprising a compound of Formula I, or salts derived thereof, and, optionally, pharmaceutically acceptable excipients and/or carriers.

The third embodiment of the present invention refers to a method for treating and/preventing any senescence-associated disease or disorder which comprises administering a therapeutically effective dose or amount of the compound or *Formula I* of the invention or a pharmaceutical composition comprising thereof along with pharmaceutically acceptable excipient or carrier.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included in the compositions of the invention and that causes no significant adverse toxicological effects to the patient.
- By "therapeutically effective dose or amount" of a composition comprising the compounds of the invention is intended an amount that, when administered as described herein, brings about a positive therapeutic response in a subject having a senescence-associated disease or disorder. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

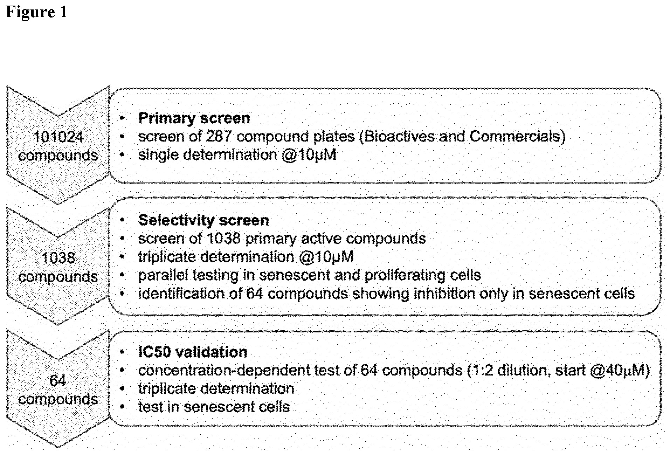

**Figure 1****. Schematic flowchart illustrating the screening strategy for identifying senolytic compounds.** Beginning with a library of 101,024 compounds, we conducted a primary screening to identify those with cytotoxic activity, resulting in 1,038 compounds. These were subsequently subjected to a selectivity screening focused on assessing cytotoxic activity specifically in senescent cells. The selectivity screening identified 64 candidate compounds, which were further subjected to IC50 validation to determine dose dependent senolytic effects.

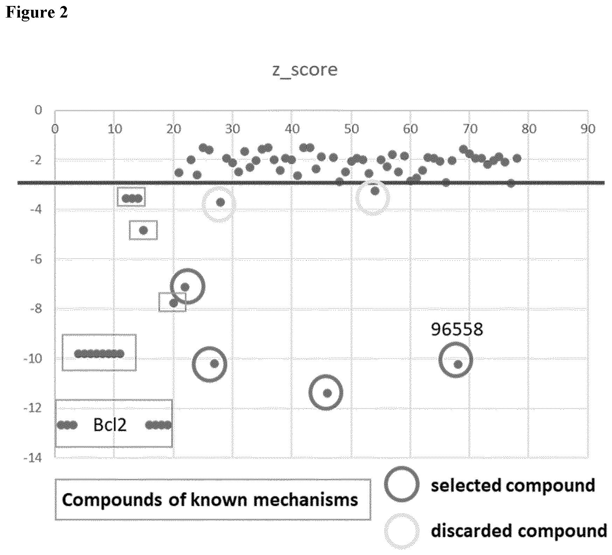

**Figure 2****. Dot plot displaying the z scores of the 64 selected compounds in the senolytic screening.** The red line denotes the threshold (z score < 3) for compounds considered to exhibit strong activity. Compounds within the orange boxes are those with known identities and activities. Compounds enclosed in yellow circles were excluded for failing the IC50 test. Compounds within the green circle represent the four most potent and validated senolytic compounds.

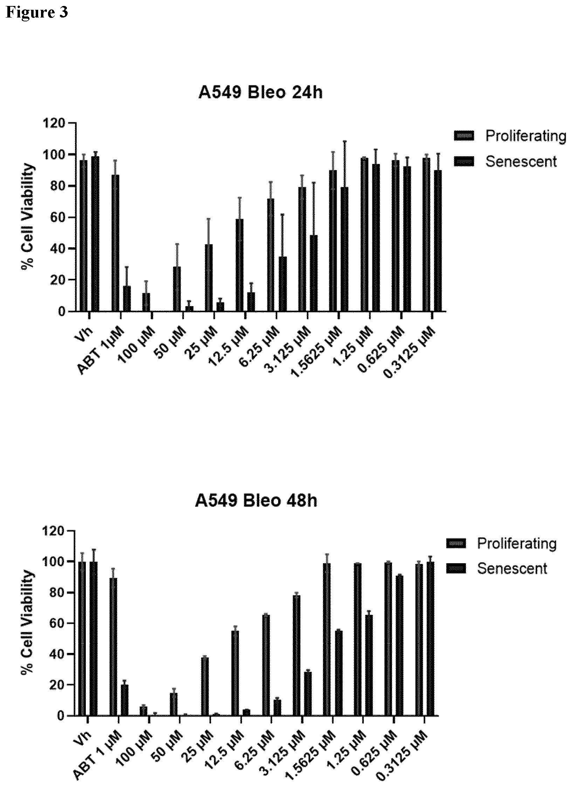

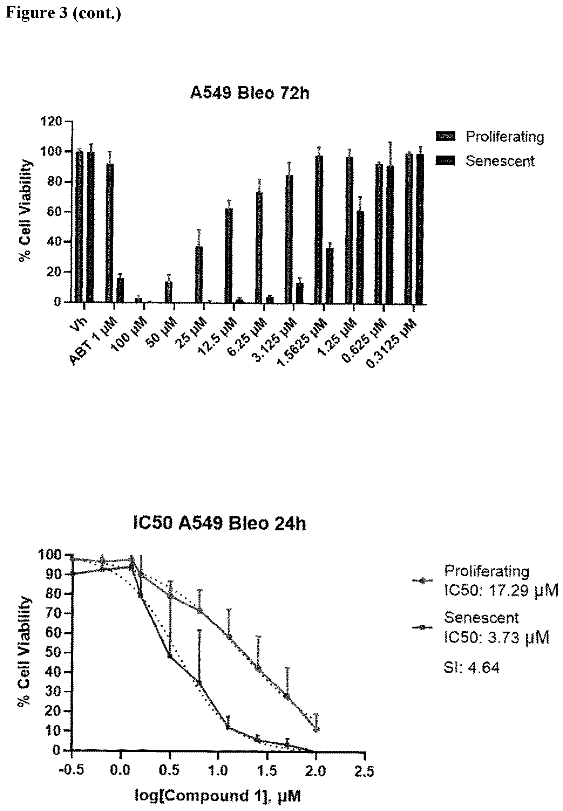

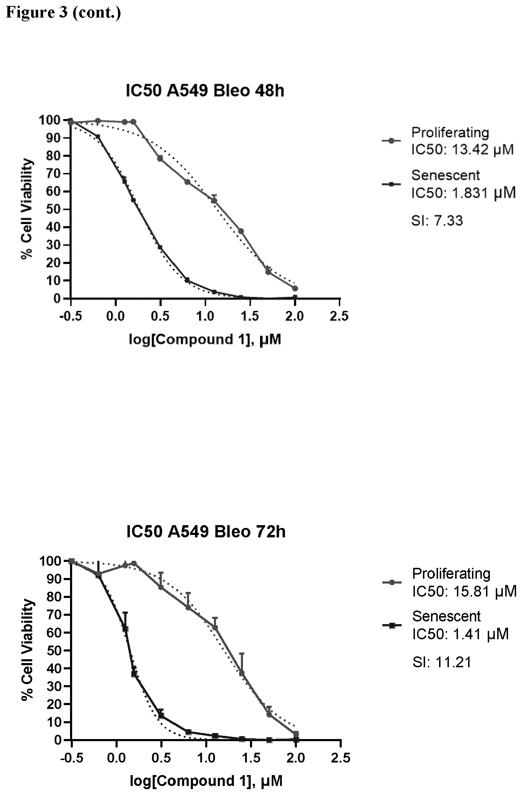

**Figure 3****. IC50 Validation of Compound 96558 in Bleomycin Induced senescent A549 cells** Cell viability graphs (upper part) and IC50 curves (bottom part) of A549 cells treated with decreasing concentrations of 96558. Senescence was induced using bleomycin, and the cell viability assay was conducted at different time points (24, 48 and 72 hours). The figure provides a visual representation of the compound's differential effects on senescent and proliferative cells, highlighting its potential as a senolytic agent.

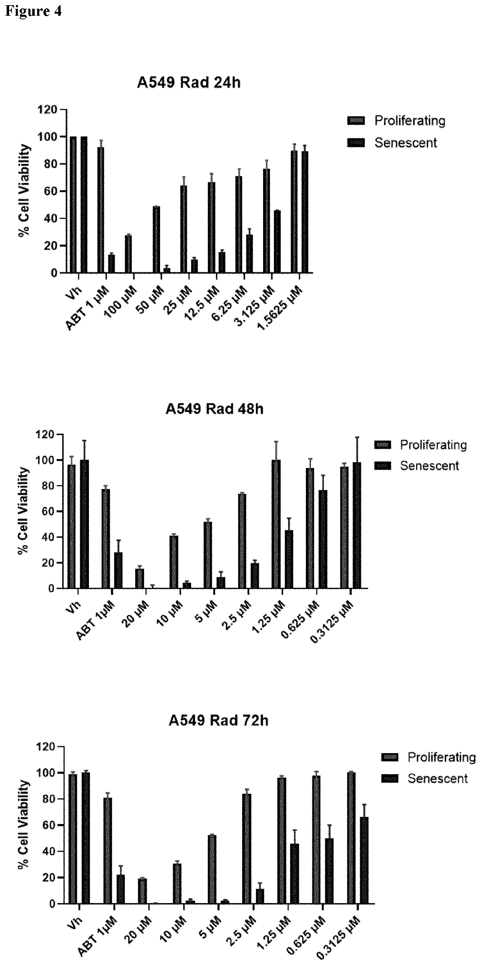

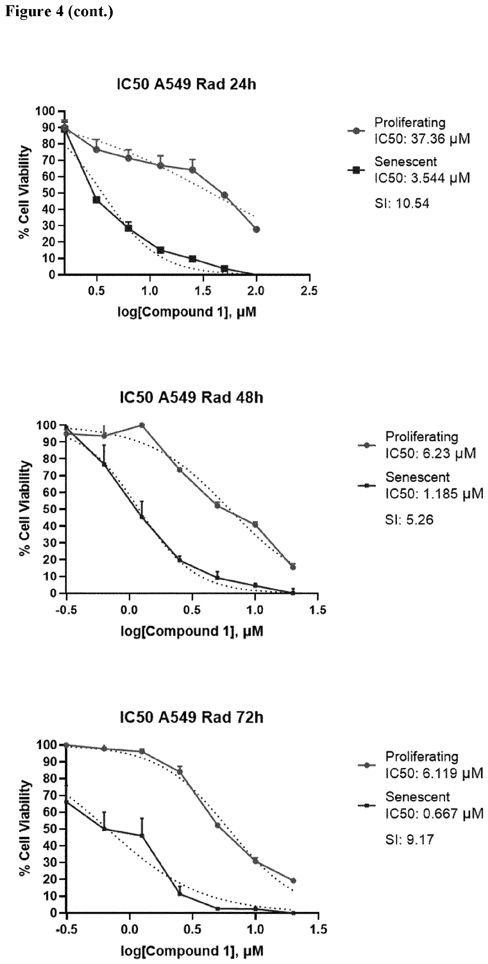

**Figure 4****. IC50 Validation of Compound 96558 in Radiation-Induced senescent A549cells.** The upper section displays cell viability graphs, while the lower section exhibits IC50 curves for A549 cells subjected to decreasing concentrations of compound 96558. Senescence was induced through ionizing radiation, and the cell viability assay was conducted at multiple time intervals (24, 48, and 72 hours). This figure visually depicts the Compound's distinct effects on senescent and proliferative cells, underscoring its potential as a senolytic agent in the context of ionizing radiation-induced senescence.

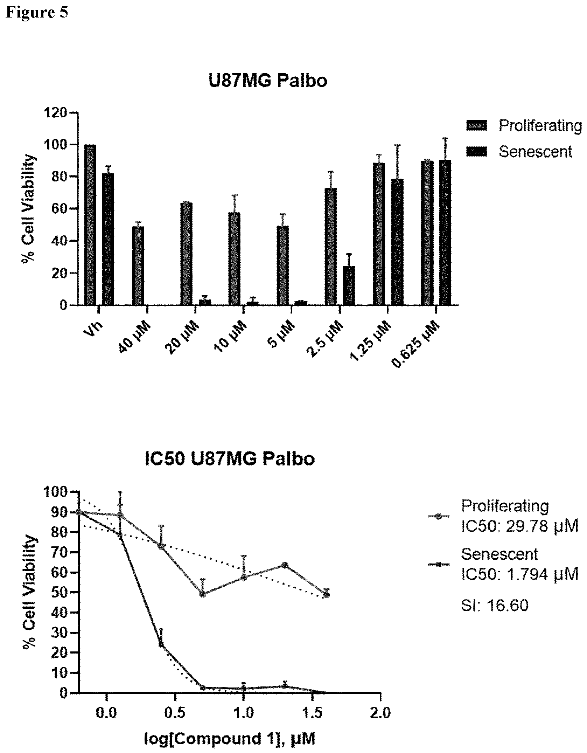

**Figure 5****. Assessment of Compound 96558's Senolytic Potential in U87MG Cells with Palbociclib -Induced Senescence.** This figure illustrates the results of evaluating the senolytic potential of Compound 96558 under an alternative chemotherapeutic condition and senescence induction method. Upper part: Cell viability graph in U87MG cells induced into senescence with palbociclib. Bottom part: IC50 curve for this cell line.

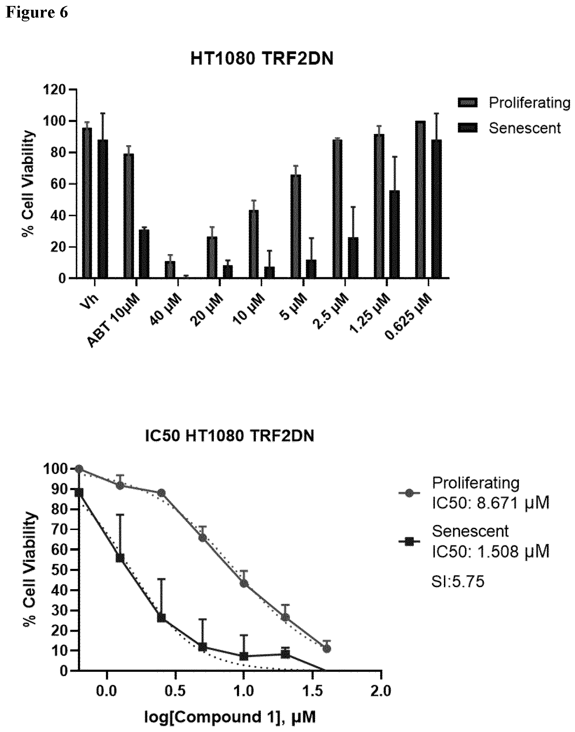

**Figure 6****. 96558's Senolytic Efficacy in HT1080 Cells with Trf2 Deletion**. The figure presents the outcomes of evaluating the senolytic potential of Compound 96558 under a different senescence induction method. Upper part: Cell viability graph in HT1080 cells induced into senescence through Trf2 deletion. Bottom part: IC50 curve for this cell line.

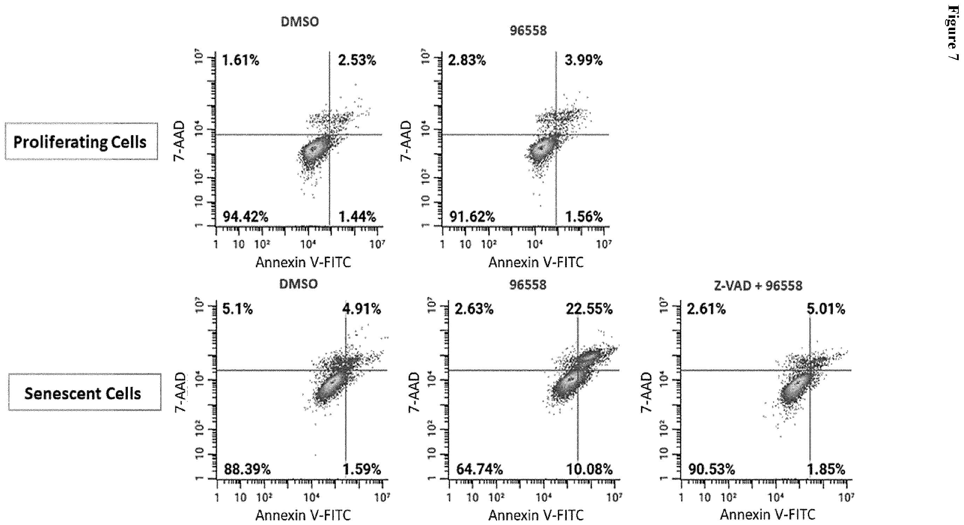

**Figure 7****. Compound 96558 induces apoptosis in A549 senescent cells.** Representative flow cytometry dot plots of A549 cells both proliferating and bleomycin induced senescent cells using Annexin V/7 AAD.

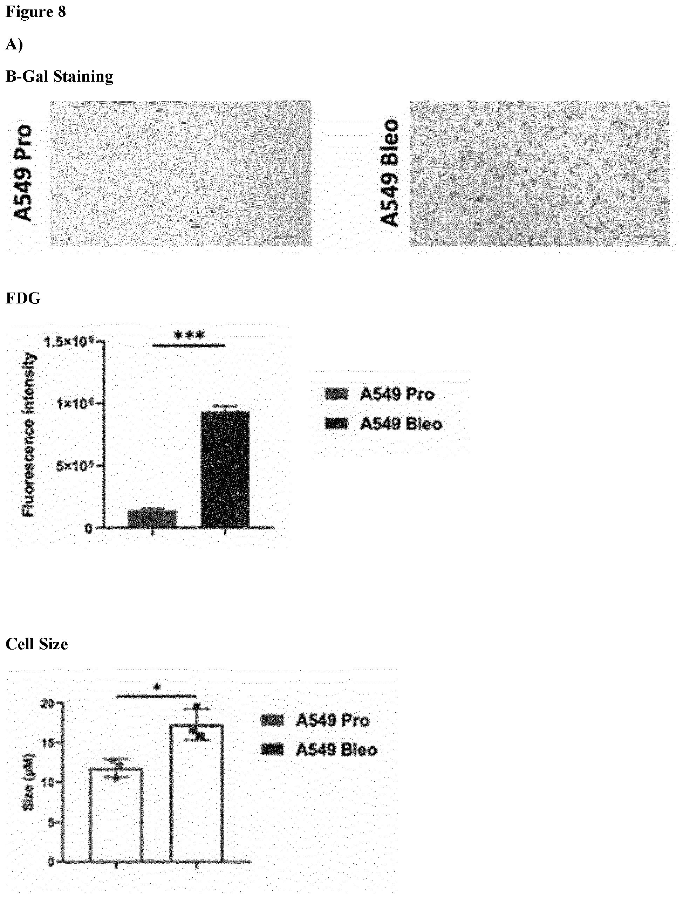

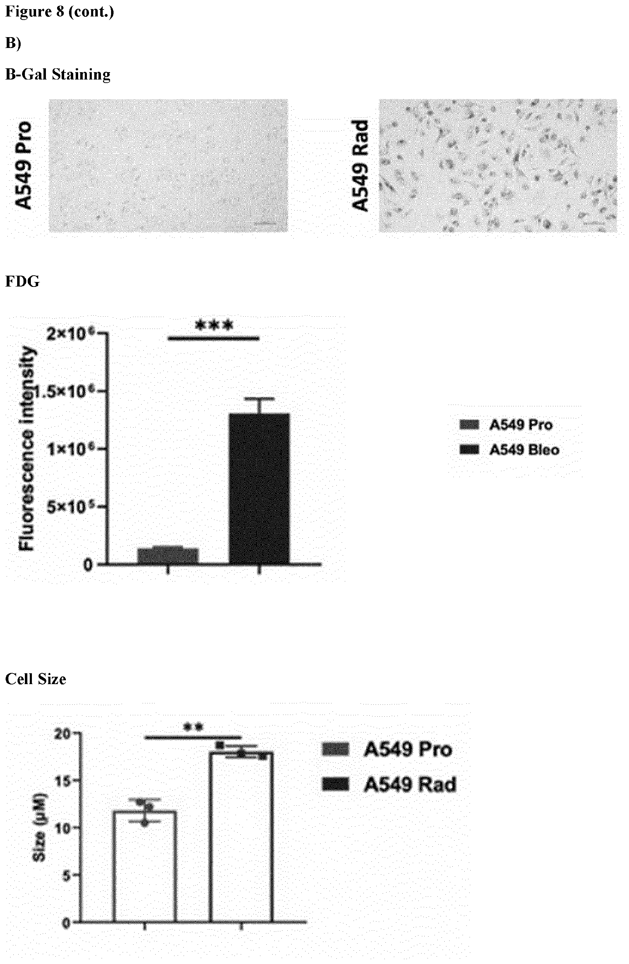

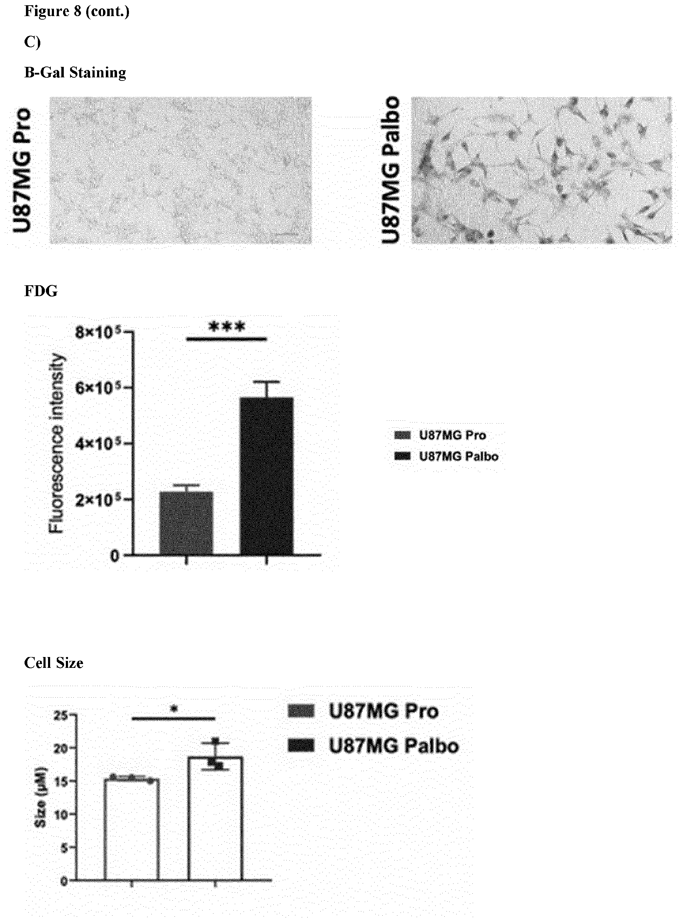

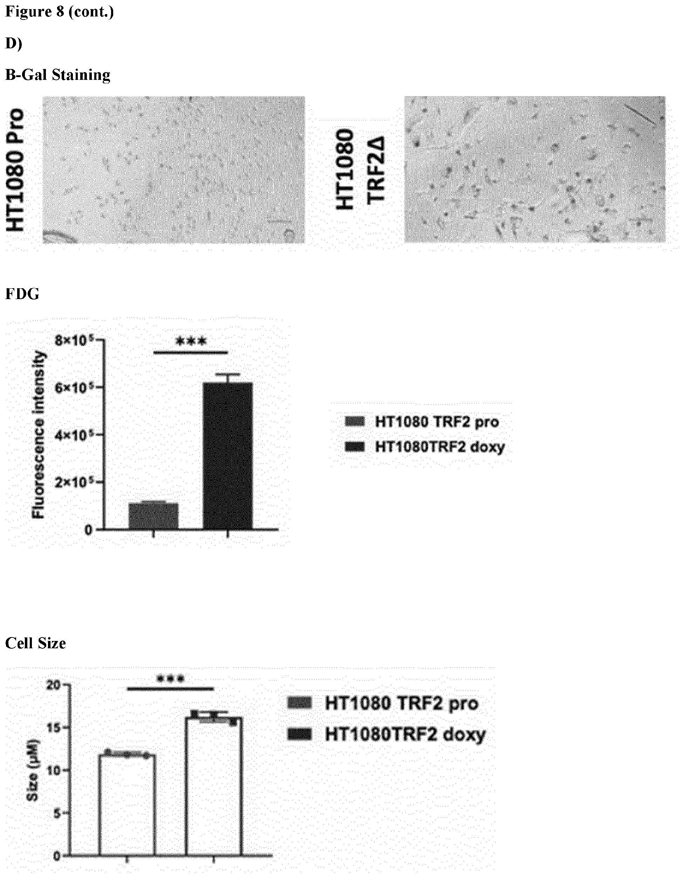

**Figure 8****. Characterization of the Senescent State.** This figure shows Beta-gal staining in senescent cells highlighted in blue, increased FDG fluorescence intensity in senescent cells and enlargement in cell size in senescent cells. The conditions include **A)** A549 proliferating cells or treated with bleomycin; **B)** proliferative or irradiated A549 cells; **C)** U87MG cells treated with palbociclib to induce senescence and proliferating ones; and **D)** HT1080 cells with TRF2 deletion, both untreated and treated with doxycycline.

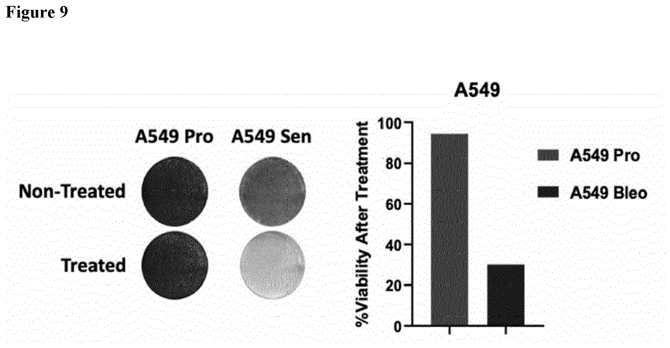

**Figure 9****. Senolytic Effect of Compound 96558 at 10 µM**. Comparison of cell viability between proliferative and bleomycin-induced senescent A549 cells. The left image displays crystal violet staining highlighting viability differences after treatment with 96558. On the right, relative absorbance at 570 nm is shown for the four conditions: proliferative cells with/without treatment (96558) and senescent cells with/without treatment (96558). Viability percentages after 96558 treatment are also provided.

### Detailed description of the invention

The present invention is illustrated by means of the Examples set below without the intention of limiting the scope of protection.

### Example 1. Material and methods

### Example 1.1. Cells and Reagents

A549 and U87MG cell lines were acquired from ATCC. HT1080 cell line carrying a DN-TRF2 gene under the tetracycline-controlled promoter was a kind gift from Dr Oscar Fernández-Capetillo (CNIO, Madrid, Spain). Cells were cultured in a controlled environment at 37°C with a 5% CO2 humidified atmosphere and were routinely tested for mycoplasma contamination. The culture medium consisted of DMEM high glucose (4500 mg/L) (Sigma) supplemented with 10% FBS (Corning), 1% glutamine (Sigma) and 1% penicillin/streptomycin solution (Sigma).

Navitoclax (ABT-263, Abbvie) was used as a positive control for senolysis. For chemotherapy-induced senescence we used Bleomycin (Mylan Pharmaceuticals) at 20 µM for 5 days. Alternatively, we also used 5 µM Palbociclib (MedChemExpress) for 7 days. Ionizing radiation at a dose of 10 Gy was applied to induce senescence. Doxycycline (Sigma), a stable tetracycline analogue, was used as an inducer for inducible expression systems at 1 µg/mL.

### Example 1.2. Crystal Violet Assay

Cell viability was tested by crystal violet staining as reported previously (Feoktistova et al. 2016). Both proliferative and senescent cells from different cell lines were plated at a concentration of 1,2 × 104 cells per well in a 96-well plate. After a 24 h incubation period, they were treated with Compound 96558 at decreasing concentrations (with DMSO as the control) and at various time points. Subsequently, cells were washed with phosphate-buffered saline (PBS, Merck) and fixed with paraformaldehyde (Electron Microscopy Science) prepared at 2% in PBS for 15 minutes, followed by a 5 min staining process using a 0.1% crystal violet solution in Milli-Q water. After staining, the excess dye was removed, washed 3 times with water, and air dried. Captured crystal violet was solubilized in 10% acetic acid and incubated on an orbital shaker at room temperature for 10 min. The absorbance was measured using a BioTek microplate reader equipped with Gen5 software.

### Example 1.3. IC50 Calculation

The half-maximal concentration of the Compound that shows effectiveness was calculated using cell cultures induced to senescence by different stimuli and subsequent treatment with the senolytic Compound starting at a high concentration and using serial dilutions. Cell viability was evaluated following the previously mentioned protocol of crystal violet staining. The IC50 calculations were performed using GraphPad Prism^{®} software.

### Example 1.4. Senescence-associated β-Galactosidase staining

Cells were fixed at room temperature using a solution containing 2% formaldehyde and 0.2% glutaraldehyde. After washing, they were incubated overnight at 37°C with a fresh SABG staining solution composed of 1 mg/mL of 5-bromo-4-chloro-3-indolyl beta-D-galactoside (X-Gal) from Fisher Scientific or 20 mM fluorescein di beta D-Galactopyranoside (FDG), along with 40 mM citric acid/sodium phosphate at pH 6.0, 5 mM K3Fe[CN]6, 5 mM K4Fe[CN]6, 150 mM NaCl, and 2 mM MgCl2. Subsequent quantification using light microscopy or fluorimetry. Cell size was determined using LUNA-IITM automated cell counter (Logos Biosystems, Anyang, South Korea).

### Example 1.5. Apoptosis Assay

A549 cells were resuspended into a single-cell suspension and plated at a density of 3 × 105 cells per well in six-well plates. After culturing for 24 h, the cells were treated with DMSO (control), the Compound, or a combination of pan-caspase inhibitor Z-VAD-FMK (Santa Cruz Biotech.) and the Compound. Following a 24 h incubation with the drugs, the cells were harvested by centrifugation at 3,000 rpm for 5 minutes and washed twice with PBS. Subsequently, the cells were resuspended in 500 µl of binding buffer. Then, 5 µl of Annexin V-FITC and 5 µl of 7-AAD (Elabscience) were used to detect the effect of Compound 96558 on cell apoptosis. The resulting samples were immediately analyzed by flow cytometry (FACScan, BD Biosciences), using the FITC channel (excitation: 488 nm and emission: 525 nm) and the 7-AAD channel (excitation: 488 nm and emission: 670 nm).

### Example 2. Results

### Example 2.1. Discovery of Novel Senolytic Compounds via High-Throughput Screening

We conducted an extensive search for potential senolytic compounds utilizing a library comprising 101,024 small molecules (EU-OPENSCREEN library). In this screening process, we employed the A549 human lung adenocarcinoma cell line, which had been subjected to a 5-day treatment with Bleomycin to induce a well-characterized senescent state. These senescent cells were plated in 384-well plates, and individual compounds from the library were added at a concentration of 10 µM, followed by a 24 h incubation period. Cell viability was assessed by quantifying cell numbers using Hoechst DNA staining on an Operetta system. A schematic representation of the screening strategy is presented in **Figure 1****.**

During the primary screening, we identified 1,038 compounds displaying cytotoxic activity in senescent cells. Subsequently, for selectivity screening, we plated senescent and proliferative A549 cells in parallel and treated them with these 1,038 compounds at 10 µM in triplicate for 24 hours. This secondary screening enabled us to select 64 compounds exhibiting specific cytotoxicity in senescent cells, indicative of their potential as senolytics.

We further assessed the senolytic activity of these 64 compounds by calculating their IC50 values. Only 15 compounds demonstrated a robust and dose-dependent senolytic effect. Notably, two of these compounds (ABT-737 and A-1155463) are known inhibitors of the BCL2 family of antiapoptotic proteins, a recognized senolytic target, thus validating the efficacy and specificity of our screening platform.

Out of these 15 compounds displaying dose-dependent senolytic activity, four exhibited significant activity. Among these four, one compound (designated as 96558 in **Figure 2**) had previously unknown senolytic activity and demonstrated a robust response in various cellular contexts.

### Example 2.2. Validation of Compound 96558 as a Senolytic Agent in A549 Bleomycin-Induced Senescent Cells

First, we sought to validate the senolytic potential of the Candidate Compound within the same cell type and under the same conditions of senescence induction as employed during the high-throughput screening. To achieve this, we used A549 cells in which senescence was induced using bleomycin (20 µM; 5 days). Subsequently, following the confirmation of the senescent status of these cells in comparison to the proliferative control (**Figure 8A**), a cell viability assay was performed, employing crystal violet staining, as detailed in the Materials and Methods section.

After evaluating the senolytic activity of the Candidate Compound at the same concentration used in the screening (**Figure 9**), we conducted further tests using a range of concentrations, from 100 µM to 0.3125 µM, in A549 tumor cells. These tests were performed both in their non-treated (proliferating) state and after the induction of senescence with Bleomycin. In addition, we also assessed the drug's ability to affect cell viability at different time points (24, 48, and 72 hours) by renewing the Compound every 24 hours.

Comparing viability, we observed how Navitoclax, our positive control, acts as a senolytic at the tested concentration, since untreated cells (proliferative ones) are barely affected (with viability close to 100%). Bleomycin-induced senescent cells, on the contrary, are significantly affected.

This same effect is found with Compound 96558 at all three measured time points and specific drug concentrations. At 24, 48, and 72 hours, using low concentrations of the Compound we could observe an efficient elimination of the senescent cells while our control, proliferative ones showed intact viability.

At 24 hours, around 12.5 µM of the drug demonstrates effective cytotoxicity in senescent cells with minimal impact on proliferative cells. As time progresses, the concentration required for this favorable senolytic effect decreases. At 48 hours, a concentration of 6.25 µM of Compound 96558 is effective, and at 72 hours, a concentration of 3.125 µM proves to be the most efficient one. Nonetheless, in all cases, the senolytic effect of the Compound is confirmed.

We determined the IC50 values at different time points, revealing lower values under senescent conditions. Specifically, at the 24-hour mark, a concentration of 3.73 µM is required to induce lethality in a minimum of 50% of the senescent cells. It is noteworthy that this concentration diminishes with extended drug incubation times, resulting in an IC50 value of 1.831 µM after 48 hours of treatment and further decreasing to 1.41 µM at 72 hours. It is important to emphasize that, across all instances, the IC50 values observed for proliferative cells are consistently higher. This underscores the Compound's greater specificity in targeting senescent cells as opposed to proliferating ones. This effect is also reflected on the fact that the senolytic index, the ratio between the IC50 values on proliferating cells over senescent ones, increases with time (**Figure 3**).

### Example 2.3. Evaluation of Compound 96558 as a Senolytic Agent under Ionizing Radiation-Induced Senescence

Subsequently, we aimed to assess the senolytic capability of our Compound using a senescence induction method different from the previous chemotherapy-induced approach. In this regard, we exposed A549 cells to ionizing radiation, confirmed the senescent status (**Figure 8B**) and conducted a similar assay as described previously. Notably, the outcomes obtained in this instance were analogous to those observed earlier, verifying the senolytic capacity in a different cellular context.

Our examination of cell viability reveals that our Compound has senolytic activity, showing a greater cytotoxic effect on senescent cells compared to their proliferating counterparts, across the 24, 48, and 72-hour treatment intervals, particularly at specific concentrations. Notably, approximately 12.5 µM of the drug at the 24-hour mark, and 2.5 µM at 48 and 72 hours, show enhanced efficacy. Furthermore, the IC50 curves illustrate that lower Compound concentrations are required for the eradication of senescent cells in comparison to their proliferative counterparts, a consistent observation across all examined conditions (**Figure 4**).

### Example 2.4. Exploring Senolytic Potential of Compound 96558 in a Different Human Tumor Context

To thoroughly explore the senolytic potential of Compound 96558, we employed a different human tumor cell line, specifically the U87MG glioma cell line. Senescence was induced through treatment with palbociclib (**Figure 8C**), thereby expanding the compound's range of potential application. This not only allowed us to test the candidate compound in a different tumor cell line but also under a different chemotherapeutic induction, enhancing the breadth of its applicability.

Once again, we conducted a viability assay to confirm the senolytic activity in this different tumoral environment. In this case, a significant difference is observed between the behavior of proliferating cells and those induced into senescence with palbociclib after exposure to Compound 96558. Senescent cells are greatly affected, with an IC50 of 1.794 µM, while a quantity of 29.78 µM is required to eliminate at least 50% of their proliferative counterparts. This results in a senolytic index of 16.6, reflecting the compound's high senolytic activity in this cellular environment.

Likewise, this IC50 analysis of the glioblastoma cell line U87MG further underscores the compound's previously proven senolytic properties (**Figure 5**).

### Example 2.5. Assessing 96558 Senolytic Efficacy in a Cellular Aging Model

To assess the senolytic efficacy of the Compound in a distinct cellular context, we turned to a genetically modified cell line of fibrosarcoma, HT1080. This particular cell line was modified for the conditional deletion of the Trf2 gene, responsible for encoding the telomeric repeat binding factor 2 (Trf2). In normal circumstances, Trf2 plays a crucial role in maintaining chromosomal stability by forming specialized structures called t-loops, which shield chromosome ends from being recognized as double-strand DNA breaks by the DNA damage repair machinery (d'Adda di Fagagna et al. 2003).

The experimental knockdown of Trf2 in HT1080 cells leads to the disintegration of the t-loop structure, an event referred to as telomere uncapping. This, in turn, triggers a rapid double-strand DNA break response, marked by p53-mediated upregulation of p21 and the induction of cellular senescence (Stansel et al. 2001). This process strikingly resembles the natural aging-related telomere shortening, which can result in critically short telomeres and subsequent uncapping (Daniali et al. 2013). Therefore, this cellular model provides valuable insights into the potential senolytic effects of the compound in a context reminiscent of cellular aging.

Similarly, we conducted an analysis of cell viability and substantiated the senolytic properties of the compound across the tested cancer cell lines, irrespective of the method employed for senescence induction or their tissue of origin. In this context, the IC50 analysis of the fibrosarcoma cell line HT1080, which was induced into senescence through the deletion of TRF2 following treatment with doxycycline (**Figure 8D**), demonstrates the compound's preferential cytotoxicity towards senescent cells in comparison to proliferating ones. When these cells were exposed to Compound 96558, the senescent cells displayed a significantly higher susceptibility to the treatment, while their proliferative counterparts exhibited a comparatively reduced response (**Figure 6**).

### Example 2.6. Apoptosis as the Main Cell Death Mechanism

To investigate the potential mechanism of cell death by which the compound eliminates senescent cells, we analyzed apoptosis induction. For this, we conducted a flow cytometry assay to evaluate Annexin-V staining on bleomycin-induced senescent cells treated or not with our senolytic Candidate. First, we confirmed that apoptosis was not induced in proliferative cells treated with vehicle (DMSO) or with Compound 96558 at 10 µM.

In senescent A549 cells, we observed a notable difference between DMSO and Compound treatment. In the latter, a significant proportion of cells were in late apoptosis, as indicated by FITC-Annexin V-positive and 7-AAD positive staining (right upper).

To further corroborate these results, we conducted a treatment with the pan-caspase inhibitor Z-VAD-FMK. The combination of Z-VAD-FMK with the drug showed a protective effect in senescent A549 cells from the senolytic cell death. In this case, the majority of cells appeared as viable cells (FITC-Annexin V and 7-AAD negative, left lower), similar to the results obtained after treatment with the DMSO control. This indicates that the senolytic effect of 96558 is mediated by the activation of the apoptotic signalling pathway (**Figure 7**).

## Claims

1. Compound of *Formula I,* or salts or derivatives thereof, 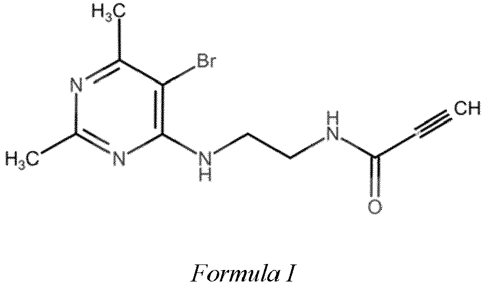 for use as a medicament.

2. Compound of Formula I, or salts or derivatives thereof, for use, according to claim 1, as senolytic agent to selectively induce death of senescent cells.

3. Compound of Formula I, or salts or derivatives thereof, for use, according to any of the claims 1 or 2, in the treatment and/or prevention of senescence-associated diseases or disorders.

4. Compound of Formula I, or salts or derivatives thereof, for use, according to any of the claims 1 to 3, in the treatment and/or prevention of senescence-associated diseases or disorders selected from: cardiovascular disorder, metabolic disease, an inflammatory disease or disorder, a pulmonary disease or disorder, a neurological disease or disorder, a proliferative disorder, a renal disorder or disease, an eye disease or disorder, and/or a dermatological disorder or disease or cancer.

5. Compound of Formula I, or salts or derivatives thereof, for use, according to any of the claims 1 to 4, in the treatment and/or prevention of senescence-associated diseases or disorders selected from: (a) an inflammatory or autoimmune disease or disorder selected from osteoarthritis, osteoporosis, oral mucositis, inflammatory bowel disease, kyphosis or herniated intervertebral disc; (b) a neurological disease or disorder selected from Alzheimer's disease, Parkinson's disease, Huntington's disease, dementia, mild cognitive impairment, macular degeneration or motor neuron dysfunction; (c) a metabolic disease selected from diabetes, diabetic ulcer, metabolic syndrome or obesity; (d) a pulmonary disease selected from pulmonary fibrosis, chronic obstructive pulmonary disease, asthma, cystic fibrosis, emphysema, bronchiectasis or age-related loss of pulmonary function; (e) an eye disease or disorder selected from macular degeneration, glaucoma, cataracts, presbyopia or vision loss; (f) an age-related disorder selected from renal disease, renal failure, frailty, hearing loss, muscle fatigue, skin conditions, skin wound healing, liver fibrosis, pancreatic fibrosis, oral submucosa fibrosis or sarcopenia; (g) a dermatological disease or disorder selected from eczema, psoriasis, hyperpigmentation, nevi, rashes, atopic dermatitis, urticaria, diseases and disorders related to photosensitivity or photoaging, rhytides, pruritis, dysesthesia, eczematous eruptions, eosinophilic dermatosis, reactive neutrophilic dermatosis, pemphigus, pemphigoid, immunobullous dermatosis, fibrohistocytic proliferations of skin, cutaneous lymphomas or cutaneous lupus; or (h) a cardiovascular disease selected from atherosclerosis, angina, arrhythmia, cardiomyopathy, congestive heart failure, coronary artery disease, carotid artery disease, endocarditis, coronary thrombosis, myocardial infarction, hypertension, aortic aneurysm, cardiac diastolic dysfunction, hypercholesterolemia, hyperlipidemia, mitral valve prolapse, peripheral vascular disease, cardiac stress resistance, cardiac fibrosis, brain aneurysm or stroke.

6. Pharmaceutical composition comprising a compound of Formula I, or salts derived thereof, 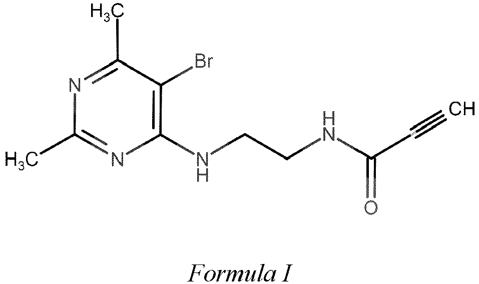 and, optionally, pharmaceutically acceptable excipients and/or carriers.
